# EUROPEAN PATENT APPLICATION

(11) **EP 1 106 171 A1**
(43) Date of publication of application: **13.06.2001**
(21) Application number: 99811117.3
(22) Date of filing: 06.12.1999
(51) Int. Cl.: A61K 7/46

(54) **Fragant formulations**

(71) Applicant: Simbürger, Isabella, 6850 Dornbirn (AT)
(72) Inventor: Simbürger, Isabella, 6850 Dornbirn (AT)
(74) Representative: Arnold, Winfried

(57) **Abstract**

The invention concerns a stable homogeneous aqueous fragrance formulation comprising a fragrance composition, a surfactant and water, and which is alcohol-free, a vessel equipped with a valve for spraying, comprising said fragrance formulation, methods for producing said fragrance formulation and said vessel, and the use of the fragrance formulations for the manufacture of a perfume, deodorant, cosmetic, aromatherapeutic, spagyric essence, wound or room disinfectant, insecticide, insect or marten repellent, and the like.

## Description

### Field of the Invention

The inventions concerns a stable, aqueous, ethanol-free fragrance formulation, a vessel equipped with a valve for spraying said formulation, and a method of producing said formulation and vessel. Said formulation and vessel are used for the manufacture manufacture of. a perfume, deodorant, cosmetic, aromatherapeutic, spagyric essence, wound or room disinfectant, insecticide, insect or marten repellent, and the like.

### State of the Art

Various fragrance formulations and vessels for spraying such formulations are known and widely used in the art of cosmetics, deodorants, room aromatizing, aroma-therapy, insect or marten repellents, and the like. These formulations contain lipophilic compounds, such as essential oils or other lipophilic, water insoluble fragrant ingredients.

Fragrance or perfume compositions in deodorants, disinfectants, insect or marten repellents, in room sprays, mouth and nose sprays, in aroma-therapy, and spagyric compositions are wellknown in the art. Representative publications include "The Illustrated Encyclopedia of Essential Oils, The Complete Guide to the Use of Oils in Aromatherapy and Herbalism", by Julia Lawless, Klement Hooks, Shaftesbury, Dorset, GB 1995 (German issue "Die illustrierte Enzyklopädie der Aromaöle", Scherz Verlag, Bern etc. 1996), "Düfte bewusst erfahren und nutzen", Erich Keller, Scherz Verlag, Bern, München, Wie, 1995, "Düfte der Natur, Heilessenzen & Aromaole", Gerti Samel and Barbara Krähmer, Publischer W. Ludwig Buchverlag in der Südwest Verlag GmbH & Co. KG, München, 1997, and "Düfte helfen Heilen, Handbuch der Aromatherapie", Ingrid Diersen:, Hallwag AG, Bern, 1997.

Aqueous mixtures of lipophilic fragrance compositions are unstable and separate into the oily and the aqueous layer. In order to avoid separation into two layers water soluble solvents and/or surface active compounds ("surfactants") as solubilisers or emulsifiers are added. Most common as solvent or solubiliser is a high percentage of a lower alcohol, in particular ethanol, or also isopropanol or glycerine, which are usually used in combination with a surfactant.

Some room deodorising or disinfecting formulations contain enzymes which help to oxidise and/or split bad smelling compounds. Such enzymes are expensive and quite often not very stable.

Single phase, homogeneous aqueous formulations in particular need an alcohol or another solubiliser, e.g. acetone, in order to maintain a clear solution or at least a homogeneous emulsion of the lipophilic fragrance comprised by the formulation. Alcohol or acetone containing fragrance formulations impose considerable risks during their production, storage, transportation and use as they are easily inflammable, in particular if sprayed in form of an aerosol and if in contact with a hot material, e. g. a hot cocking-plate, a hot automobile motor, smoulder cigarette or burning candle. Similarly other organic solvents, in particular if they have low boiling points, are likewise easily inflammable, especially if finely dispersed in an aerosol. Most fragrance composition, such as essential oils have a high vapour pressure and are by themselves inflammable.

A disadvantage of the most commonly used ethanol in fragrance formulations for animal or human use is that it may irritate sensitive skin and cause a painful reaction. The use of ethanol in the aerosol furthermore has the other disadvantage in that it may anaesthetise the olfactory nerves. The loss of fragrance intensity may only be counteracted by a higher concentration of the fragrant ingredients. This has economic consequences. Alcohol also increases the addictive potential which is to be avoided in particular in hospitals, schools and kindergartens.

In order to lower the alcohol content of frangrance formulations addition of one or more surfactants is common in the art. Such surfactants are mainly selected from those also used and acceptable in pharmaceutical and cosmetical formulations and include for example those of the polyethyleneglykol type.

An alcohol-free aerosol formulation is described in US 5,508,023. The formulation comprises less than 5% by weight of micronized drug, less than 5 % by weight of at least one polar surfactant, and 90 % by weight of 1,1,1,2-tetrafluoroethane or 1,1,1,2,3,3,3-heptafluoropropane. The fluoroethane or -propane is used as solvent and propellant. This formulation is quite expensive, ecologically unsatisfactory and does not contain water.

The prior art of fragrance formulations has not yet found a solution for avoiding alcohol in aqueous formulations. In contrast, there exists still the conviction that alcohol is an absolutely necessary ingredient Stable, aqueous fragrance formulations, such as aerosol formulations, comprising lipophilic fragrance compositions in form of homogeneous solutions or emulsions, and free of alcohol have not become known.

### Objective of the Invention

It is an objective of the invention to provide a new fragrance formulation which lacks the disadvantages of the formulations hitherto known. It needs to meet a number of criteria. The new fragrance formulation should be alcohol-free, enzyme-free, physically and chemically stable, i. e. homogeneous and not separating into two layers, under normal and higher or lower temperatures. It should be easily sprayable. The aerosol produced by this formulation upon spraying should not be inflammable, should be non-toxic, cosmetically and pharmaceutically acceptable to the skin and sensitive organs, should not have addictive properties and should need a lower amount of the expensive fragrant ingredients.

It is an object of the present invention to solve the above problems. Provided are new fragrance formulations which lack the disadvantages of prior art formulations, which are surprisingly and unexpectedly extremely stable at room temperature, and even at lower and higher temperatures, and which are not inflammable. This goal is achieved by the present invention described in more detail in the following.

### Detailed Description of the Invention

The invention concerns a stable aqueous fragrance formulation, characterised in that it comprises a fragrance composition, water and a surfactant, said formulation being alcoholfree.

The fragrance composition comprised by the fragrance formulation is any fragrance composition known in the art, such as a fragrant perfume, an aroma-therapeutic, an air hygienic agent, an insect or marten repellent and the like. Such compositions are composed of natural or synthetic fragrant compounds. They are usually of lipophilic nature. The term comprises essential oils, fragrant alcohols, esters, ethers, aldehydes, and/or acetals common in the art of perfume compositions or spagyric compositions.

The fragrant ingredient may be a naturally occurring essential oil, such as rose oil, bergamot oil, jasmine oil, peppermint oil, rosemary, camomile, lavender, marjoram, and the like oil. An animal fragrant, is for example musk, castoreum, aber or zibet. Spagyric essences are also known in the art. They are made by fermenting certain herbs, which were collected while they were flowering, in the presence of water and yeast, steam distilling off the active ingredients, and concentrating the distilate to the spagyric basic essence. The remaining mash is calcinated, and the basic essence and the calcinated mash are combined to give the final spagyric essence. Synthetic fragrant ingredients are for example synthetic essential oils, such as composed of single compounds, such as linalol, terpineol, nerol, citronelal, benzaldehyde, cinnamon aldehyde, vanillin, methylacetophenone, and the like. Comprised are also mixtures of such ingredients.

The fragrance formulation may have, in addition to the desired pleasant smell, disinfectant activities, such as desirable in room sprays for hospitals, kitchens and toilettes, or also in mouth or nose sprays. The disinfectant effect may directed against virus, bacteria or yeasts. The fragrance formulation may have a therapeutic effect as known in aromatherapy. Well known are stimmulating, antidepressive or narcotic effects of certain fragrance compositions. In this case it comprises one or more essential oils, in particular of natural origin and having the desired therapeutic effect. A therapeutic effect may also be provided by spagyric essences, which are mostly also of fragrant nature. Such fragrance formulations may be applied in form of room, mouth or nose sprays.

The fragrance formulation may also have an insect or marten repellent effect or be useful in plant protection.

The fragrant composition is present in amounts of less than about 20%, preferably of less than about 10% by weight or volume. In general, a sufficient or even excellent fragrant smell is surprisingly obtained with a relatively low amount, e. g. about 0.01 to about 3% of the fragrant composition. This amount is lower than in common fragrance formulations, however provides a similar or even stronger fragrance intensity.

The surfactant used in the present fragrance formulation is one known in the art and is cosmetically or pharmaceutically acceptable. Preferably it is selected from the group consisting of one or more polyoxyethylene sorbitan fatty acid esters, monopolyoxyethylene alkyl ethers and monopolyoxyethylene alkanoic acid esters.

Polyoxyethylene sorbitan fatty acid esters are for example selected from the group of watersoluble or -dispersible Tweens, such as polyoxyethylene sorbitan monolaurate (Tween 20), polyoxyethylene sorbitan monostearate (Tween 60) or polyoxyethylene sorbitan monooleate (Tween 80).

A preferred group of surfactants is represented by the general formula

R-O-CH₂(CH₂OCH₂)ₙ CH₂OH I,

wherein R is an alkyl group of the partial formula CH₃(CH₂)ₘ- or an acyl group of the partial formula CH₃(CH₂)ₘ₋₁-CO-, wherein m is of from 9 to 19 and n is from 1 to 99.

If R is an alkyl group n is preferably 1 to 24. If R is an acyl group n is preferably 29 to 99. The index m is preferably 9, 11, 13, 15, 17, or 19. The index n is preferably 1, 3, 9, 17, 19, 21, 29, 39, 39, 49 or 99. Higher values of n may be only proximate or average values.

Preferred are commercially available ethers of the formula I, e. g. known under the proprietary names AKYPOROX^{TM} RLM 22, RLM 40, RLM 80, CO 400, Brij^{TM} 30, 35, 52, 56, 58, 72, 76, 78, 92, or 96, CETOMACROGOL^{TM} 1000, EMULGIN^{TM} 700 or 1000, or esters of the formula I, e. g. MYRJ 45, 49, 51, 52, 52 C, 53, or 59.

Particularly preferred surfactants of the formula I are tetrapolyoxyethylene monododecyl (lauryl) ether, tetrapolyoxyethylene monotetradecyl (myristyl) ether and mixtures thereof, such as present in the ether from tetrapolyoxyethylene and coconut alcohol, e. g. AKYPOROX^{TM} RLM 40.

The surfactant is present in the fragrance formulation in an amount sufficient to provide a stable, homogeneous, monophasic aqueous solution or emulsion. The amount depends mainly on the amount and lipophilic nature of the fragrance composition in the formulation. In general the ratio of fragrance composition to surfactant is from about 1:0.01 to about 1:5 either by weight or by volume. Whenever in the present description ratios or percentages are given they are either by weight or by volume if not specifically defined.

If for example the amount of fragrance composition is about 1%, the amount of surfactant is about 0.5%. In general, less than from about 3% to about 10% of surfactant is sufficient to provide a homogeneous stable formulation.

The water in the aerosol formulation may be normal tap water, or distilled or deionised water, preferably with a pH of about 7. It may be necessary to adjust the entire aerosol formulation to a pH around 7.

Such fragrance formulation comprises e. g. 3% of fragrant composition, 3% of surfactant and 94% of water, or 1% of fragrant composition, 0.5% of surfactant and 98.5% of water.

The present fragrance formulations lack any large amounts of easily inflammable ingredients, such as ethyl alcohol, isopropanol, acetone, ethers, lower carbon hydrogens and the like, which are usually present in common aerosol sprays.

Although most of the fragrance compositions, in particular the essential oils, are easily inflammable by themselves, they are surprisingly not inflammable in the present aqueous fragrance formulations. This may be so in view of the low concentration and the presence of comparative large amounts of water.

Advantageously the present fragrance formulation is enzyme-free, thus limiting the risk of allergic reactions and enzymatic destruction of the organic ingredients in the formulation.

The invention concerns further a fragrance formulation, as described hereinbefore inserted together with a propellant in a spray vessel equipped with a valve.

The present fragrance formulation is preferably sprayable by forcing through a tiny outlet under applying sufficient pressure. For that purpose the formulation devices able to perform this task are well known in the art and comprise closed vessels which can be set under pressure by a propellant and to which a valve with a fine outlet is attached which upon operating releases a preselectable amount of the composition in form of an aerosol.

The invention concerns further a vessel equipped with a dosage valve, characterised in that it comprises a stable aqueous fragrance formulation as described hereinbefore and a propellant.

The valve is not metered or preferably a metered dose provider, standardised to eject by each push a specific amount, e.g. from 0.1 to 0.5 cm³ of the fragrance formulation. If desired the vessel is equipped with a valve which is standardised to eject in a preselected time a standardised amount of aerosol.

The propellant is not inflammable and is selected from one common in the art, e. g. from the group consisting of a fluorinated hydrocarbon, such as 1,1,1,2,3,3,3-heptafluoropropane (227) or in particular 1,1,1,2-tetrafluoroethane (134a), or air, nitrogen, or nitrous oxide.

The vessel is made of a non-corrosive metal, such as tin, a metal being protected against corrosion by a hard and insoluble organic polymer, or is made of glass or a hard and insoluble polymer.

The invention concerns further a method for producing a fragrance formulation according to the invention, characterised in homogeneously mixing the fragrant composition, the surfactant and the water. Homogeneously mixing is achieved by vigorously shaking, stirring, vibrating or sonicating the mixture with a common mixing device at a temperature of between about between 10° C and 50° C, e.g. at room temperature.

The mixing time depends mainly on the amount of the ingredients. Small amounts, e. g. to provide about 10 to about 500 ml of fragrance formulation, are homogeneously mixed within about one minute or less.

Preferably the fragrance composition and the surfactant are at first homogeneously mixed to give a more or less sticky fluid, whereupon the water is added, e. g. slowly or by drops.

The invention is further directed to the use of the stable, aqueous, alcohol free fragrance formulation for the manufacture of a perfume, deodorant, such as an arm spray (deos), cosmetic, aromatherapeutic, spagyric essence, wound or room disinfectant, insecticide, insect or marten repellent, and the like. These products are in form of solutions or emulsions, for example filled in roller bottles, or in particular filled in spray vessels, for use as sprays, such as room sprays, arm, mouth or nose sprays, and form easily aerosols.

The following examples serve to further characterise the invention, however, should not be construed as a limitation thereof.

### Example 1. General Procedure

1.0% by volume of fragrance composition
0.5% by volume of surfactant
98.5% by volume of water

At room temperature (about 23°C) the fragrance composition and the surfactant are homogeneously mixed by shaking or stirring. About 1 to 2% of the water is added and again stirred to give a homogeneous sticky fluid. The rest of the water is added and stirring continued until a homogeneous solution or emulsion is obtained.

The solution or emulsion may be filled into a bottle with a normal or safety stopper cap. Alternatively, if an aerosol is intended, the bottle may be equipped with a standardised metered valve allowing aerosol release of 0.1 cm³ upon one push. The propellant is for example air.

The solution or emulsion may also be filled into a pressure bottle equipped with a valve which may be standardised and metered. The propellant, one common in the art, usually 1,1,1,2,-tetrafluoroethane, is added and the bottle sealed. The valve may be metered in that it allows an aerosol release of 0.086 cm³ upon each push.

The bottles may have a capacity of about 5 to 500 cm³.

### Example 2. A fragrance formulation for a room deodorant

A mixture of 3 ml of a fragrance composition consisting of the following essential oils
3 ml of cedar wood,
3 ml of petit grain bigarade,
3 ml of petit grain mandarinnier,
2 ml of lavender,
7 ml of blood orange, and
1 ml of elemi,

3 ml of the surfactant AKYPOROX^{TM} RLM 22, RLM 40, and 94 ml of water (tap water, distilled or deionised) are emulsified at about 25 ° C according to Example 1 until a complete emulsion is obtained.

The emulsion is filled into a pressure bottle equipped with a standardised valve allowing aerosol release of 0.086 cm³ upon one push.

The emulsion is stable within a temperature range of at least -5° C to 50° C over a time of at least 12 month.

### Example 3. A fragrance formulation for a room deodorant

A mixture of 10 ml of a fragrance composition consisting of the following essential oils
1 ml of carrot seeds
5 ml of grape fruit
3 ml of litsea
4 ml of lemongrass
1 ml of lavender
1 ml of mountain pine
6 ml of lemon
1 ml of elemi, and 2 ml of mandarin yellow,

5 ml of the surfactant AKYPOROX^{TM} RLM 22, RLM 40 and 85 ml of water (tap water, distilled or deionised) are emulsified at about 25 ° C in analogy to Example 1 until a complete emulsion is obtained.

The emulsion is filled into a pressure can equipped with a standardised valve allowing aerosol release of 0.086 cm³ upon one push.

The emulsion is stable within a temperature range of at least -5° C to 50° C over a time of at least 12 month.

The invention has been described in terms of its best mode and embodiments. Nevertheless those skilled in the art will recognise that the invention can be practised with equivalent modifications within the spirit and scope of the appended claims.

## Claims

1. A stable aqueous fragrance formulation, characterised in that it comprises a fragrance composition, water and a surfactant, said formulation being alcohol-free.

2. The fragrance formulation according to claim 1, wherein the fragrance composition is a fragrant perfume, optionally with an aromatherapeutic or an air hygienic activity, or a mixture thereof.

3. The fragrance formulation according to claim 2, wherein the perfume is composed of a fragrant essential oil, higher alcohol, ester, aldehyde, ether, acetal, or an aqueous flower extract, or a mixture thereof, which is common in fragrance compositions or in spagyric compositions.

4. The fragrance formulation according to claim 2, wherein the fragrance composition is of natural or synthetic origin.

5. The fragrance formulation according to claim 2, wherein the fragrance composition is a room aromatizer.

6. The fragrance formulation according to claim 2, which does not contain any easily inflammable ingredients, such as ethyl alcohol, isopropanol, acetone, lower hydrocarbons or the like, or enzymes.

7. The fragrance formulation according to claim 2, wherein the fragrance composition is present in amounts of less than about 20 %.

8. The fragrance formulation according to claim 2, wherein the fragrance composition is present in amounts of less then about 10 %.

9. The fragrance formulation according to claim 2, wherein the fragrance composition is present in the amount of 0.01 to about 3%.

10. The fragrance formulation according to anyone of claims 1 to 9, wherein the surfactant is selected from the group consisting of one or more polyoxyethylene alkyl ethers, polyoxyethylene alkanoic acid esters and polyoxyethylene sorbitan fatty acid esters..

11. The fragrance formulation according to claim 10, wherein the surfactant is represented by the general formula
R-O-CH₂(CH₂OCH₂)ₙ CH₂OH I,
wherein R is an alkyl group of the partial formula CH₃(CH₂)ₘ₋ or an acyl group of the partial formula CH₃(CH₂)ₘ₋₁-CO-, wherein m is of from 9 to 19, and n is from 1 to 99.

12. The fragrance formulation according to claim 11, wherein n is preferably 1 to 24 if R is an alkyl group, or n is preferably 29 to 99 if R is an acyl group,

13. The fragrance formulation according to claim 11, wherein m is 9, 11, 13, 15, 17, or 19, and n is 1, 3, 9, 17, 19, 21, 29, 39, 39, 49 or 99.

14. The fragrance formulation according to claim 11, wherein the surfactant is mono-tetrapolyoxyethylene dodecyl (lauryl) ether, mono-tetrapolyoxyethylene tetradecyl (myristyl) ether or a mixture thereof, such as present in the mono-ether of tetrapolyoxyethylene and coconut alcohol.

15. The fragrance formulation according to claim 1, wherein the ratio of fragrance composition to surfactant is from about 1:0.01 to about 1:5, and in particular about 1:0.5.

16. The fragrance formulation according to claim 10, wherein the surfactant is present in the amount of about 1.5 % by weight or volume.

17. The fragrance formulation according to claim 10, wherein the surfactant is the mono ether of tetrapolyoxyethylene and coconut alcohol and is present in the amount of about 0.5 to about 3% by weight or volume.

18. The fragrance formulation according to claim 1, which comprises 1 % by weight or volume of fragrance composition, 0.5 % by weight or volume of surfactant and 98.5 % by weight or volume of water.

19. The fragrance formulation according to claim 1, inserted together with a propellant in an aerosol vessel equipped with a valve.

20. A vessel equipped with a valve, comprising a fragrant formulation according to claim 1.

21. A vessel according to claim 20, wherein the propellant is not inflammable and is selected from the group consisting of a fluorinated hydrocarbon, such as 1,1,2,3,3,3-heptafluoroheptan or 1,1,1,2,-tetrafluoroethane.

22. A vessel according to claim 20, wherein the propellant is air, nitrogen, or nitrous oxide.

23. A vessel according to claim 20, wherein the valve is standardised to eject from 0.1 to 0.5 cm³ of the fragrant formulation in form of an aerosol.

24. A vessel according to claim 20, wherein the valve is standardised to eject in a preselected time a standardised amount of the fragrant formulation in form of an aerosol.

25. A vessel according to claim 20, which is made of a non-corrosive metal, such as tin, a metal being protected against corrosion by a protecting hard and insoluble organic polymer, or is made from glass or a hard and insoluble polymer.

26. A method for producing a fragrance formulation according to claim 1, characterised in mixing the fragrance composition with the surfactant, then adding the water and homogenising the mixture with the aid of a common mixing device in a conventional manner.

27. Use of the fragrance formulation according to claim 1 for the manufacture of a perfume, deodorant, such as an arm spray (deos), cosmetic, aromatherapeutic, spagyric essence, wound or room disinfectant, insecticide, insect or marten repellent, and the like.
